# EUROPÄISCHE PATENTSCHRIFT

(11) **EP 4 164 553 B1**
(45) Veröffentlichungstag und Bekanntmachung des Hinweises auf die Patenterteilung: **25.10.2023**
(21) Anmeldenummer: 21732870.7
(22) Anmeldetag: 11.06.2021
(51) Int. Cl.: A61F 2/58, A61F 2/76

(54) **PROTHESENHAND**
PROSTHETIC HAND
PROTHÈSE DE MAIN

(30) Priorität: 12.06.2020 DE 102020115575
(43) Veröffentlichungstag der Anmeldung: 19.04.2023
(73) Patentinhaber: Otto Bock Healthcare Products GmbH, 1110 Wien (AT)
(72) Erfinder: LEDINGER, Christoph, 2514 Möllersdorf (AT); SAGMEISTER, Luis, 2823 Pitten (AT)
(74) Vertreter: Gramm, Lins & Partner Patent- und Rechtsanwälte PartGmbB
(86) Internationale Anmeldenummer: PCT/EP2021/065760
(87) Internationale Veröffentlichungsnummer: WO 2021/250233

(56) Entgegenhaltungen:
- DE-A1-102017 005 761
- DE-U1-202017 000 172
- US-A1- 2019 183 661

## Beschreibung

Die Erfindung betrifft eine Prothesenhand mit einem Chassis und zumindest einem Basiselement zur Befestigung eines Prothesenfingers an dem Chassis.

Prothesenhände dienen zur prothetischen Versorgung einer oberen Extremität. An einem Unterarmschaft oder einem Unterarmrohr ist eine Prothesenhand angeordnet, um zumindest eine Funktion einer natürlichen Hand nachzubilden, um einen Patienten mit einer erweiterten Funktionalität auszustatten. In der Regel ist diese Funktionalität eine Greiffunktion. Dazu ist an einem Chassis oder einem Grundkörper ein Greifelement beweglich gelagert, das durch eine Antriebseinrichtung bewegt werden kann. Neben einer mechanischen Betätigung, beispielsweise über ein Zugmittel, das durch eine Schulterbewegung aktiviert wird, sind in modernen Protheseneinrichtungen Greifelemente oder Prothesenfinger über zumindest einen motorischen Antrieb angetrieben an einem Chassis gelagert. Die Greifeinrichtungen oder Prothesenfinger selbst können gelenkig ausgebildet sein, um eine Funktionalität bereitzustellen, die der eines natürlichen Fingers angenähert ist. Ein oder mehrere Fingergelenke können in den Prothesenfingern ausgebildet sein, sodass bei einer Schließbewegung oder einer Bewegung der Finger zur Handinnenfläche hin diese flektiert werden. Überwiegend werden die Prothesenfinger an ihren proximalen Enden um eine Schwenkachse verlagerbar an dem Chassis gelagert.

Die jeweiligen Antriebe zur Verlagerung der Prothesenfinger relativ zu dem Chassis oder der Fingerelemente zueinander befinden sich häufig in dem Chassis, das in seiner Grundform an die Handfläche einer natürlichen Hand erinnert. Die Prothesenfinger werden an dem Chassis auf unterschiedliche Arten und Weisen befestigt, überwiegend über ein Scharniergelenk, das an dem Chassis ausgebildet ist. Eine Achse wird durch zwei Lagerböcke an dem Finger oder dem Chassis gesteckt und verriegelt diese in einem Lagerzapfen oder eine Lagerscheibe an der anderen Komponente. Sowohl hinsichtlich der Montierbarkeit als auch der Demontierbarkeit der einzelnen Prothesenfinger sind solche Anordnungen problematisch. Darüber hinaus ergeben sich Schwierigkeiten der Abdichtung sowie der Haltbarkeit.

Prothesenhände aus dem Stand der Technik sind beispielsweise in der DE 10 2014 005 528 A1, der US 2014/107805 A1 oder der US 2013/305984 A1 beschrieben.

Die US 2019/0183661 A1, die den Oberbegriff des Anspruchs 1 offenbart, betrifft eine angetriebene Daumenprothese mit einer Halterung und einem Finger, der um eine erste Achse und eine zweite Achse drehbar an der Halterung angeordnet, wobei die erste Achse nicht parallel zur zweiten Achse verläuft. Ein Aktuator ist so konfiguriert, dass er die Drehung des Fingers um die erste Achse bewirkt, wobei sich eine Ausrichtung der ersten Achse relativ zur Halterung ändert, wenn sich der Finger um die zweite Achse dreht.

Die DE 10 2017 005 761 A1 offenbart ein Grundglied für ein Fingerelement. Das Grundglied weist eine linke Wand und eine rechte Wand auf, welche ausgebildet sind, eine Schwenkachse zum Schwenken des Fingerglieds relativ zum Grundglied zu tragen. Das Grundglied weist eine obere Wand auf, welche ein inneres Volumen zumindest teilweise begrenzt.

Die DE 20 2017 000 172 U1 betrifft einen Handprothesengrundkörper mit einer Außenseite, einem Motor und einer ersten drehbaren Welle, die mit einer Ausgangswelle des Motors verbunden ist. Die erste drehbare Welle weist ein Kopplungselement zur lösbaren Kopplung zumindest eines Fingerelements mit der drehbaren Welle auf.

Aufgabe der vorliegenden Erfindung ist es, eine Prothesenhand bereitzustellen, bei der die Prothesenfinger einfacher zu montieren und auszutauschen sind.

Erfindungsgemäß wird diese Aufgabe durch eine Prothesenhand mit den Merkmalen des Hauptanspruches gelöst. Vorteilhafte Ausgestaltungen und Weiterbildungen der Erfindung sind in den Unteransprüche, der Beschreibung sowie den Figuren offenbart.

Die Prothesenhand mit einem Chassis und zumindest einem Basiselement zur Befestigung eines Prothesenfingers an dem Chassis sieht vor, dass an dem Chassis eine Längsführung für das Basiselement angeordnet oder ausgebildet ist, in der das Basiselement gelagert ist, wobei die Längsführung zwei translatorische Freiheitsgrade sperrt und der Längsführung ein Spannelement zugeordnet ist, das zwischen einer Verriegelungsstellung und einer Entriegelungsstellung verlagerbar in oder an dem Chassis oder dem Basiselement gelagert ist und eine Sperrung des dritten translatorischen Freiheitsgrades in der Verriegelungsstellung bewirkt. Eine Längsführung wird durch eine Nut und eine Feder ausgebildet, die korrespondierende Querschnitte aufweisen und an denen unterschiedliche Komponenten angeordnet oder ausgebildet sind, also die Nut an dem Chassis und die Feder an dem Basiselement oder umgekehrt. Durch die Anordnung des Basiselementes in einer Längsführung mit nur einem translatorischen Freiheitsgrad und einem Spannelement, das den dritten translatorischen Freiheitsgrad in der Verriegelungsstellung des Spannelementes sperrt, ist es möglich, das Basiselement und damit auch den Prothesenfinger, der an dem Basiselement befestigt, insbesondere drehbar gelagert ist, schnell und einfach zu montieren und zu demontieren. Dazu ist lediglich die Betätigung eines Spannelementes notwendig, das als ein bereits vormontierter Teil entweder an dem Chassis oder der Fingereinheit aus Basiselement und Prothesenfinger angeordnet sein kann. Das Spannelement wirkt auf das Basiselement ein und sperrt den dritten translatorischen Freiheitsgrad oder gibt ihn wieder frei. Somit muss nur das Spannelement aus der Verriegelungsstellung in die Entriegelungsstellung oder umgekehrt bewegt werden, um einen Prothesenfinger von der Prothesenhand entfernen oder daran festzulegen zu können. Es müssen keine weiteren Bauteile demontiert werden, um das Basiselement und den Prothesenfinger zu montieren oder zu demontieren. Insbesondere wenn das Spannelement in dem Chassis oder in dem Basiselement beweglich oder verlagerbar befestigt ist, kann dieses auch im demontierten Zustand nicht verloren gehen, das Spannelement ist damit ein Bestandteil des Basiselementes oder des Chassis und verbleibt an diesem im demontierten Zustand.

Eine Weiterbildung der Erfindung sieht vor, dass das Spannelement auf zumindest ein Sperrelement einwirkt und dieses aus einer Entriegelungsstellung in eine Verriegelungsstellung bringt. Über das Spannelement, das betätigbar an dem Chassis oder dem Basiselement gelagert oder befestigt sein kann, wird auf ein Sperrelement eingewirkt, das letztendlich die Relativverlagerung zwischen dem Basiselement und dem Chassis unterbindet. In einer Stellung des Spannelementes, in der Entriegelungsstellung, befindet sich auch das Sperrelement in der Entriegelungsstellung, wird das Spannelement in die Verriegelungsstellung gebracht, verlagert es das Sperrelement aus der Entriegelungsstellung in die Verriegelungsstellung und fixiert das Basiselement in der Längsführung an dem Chassis.

Das Spannelement oder das Sperrelement kann das Basiselement in der Längsführung klemmen, beispielsweise indem die Feder, die an dem Chassis oder dem Basiselement ausgebildet sein kann, innerhalb der Nut gegen eine Nutwand gepresst oder geklemmt wird. Alternativ oder ergänzend kann eine formschlüssige Verriegelung des Spannelementes oder des Sperrelementes erfolgen, sodass das Basiselement in der Längsführung verriegelt ist. Beispielsweise kann in einer Nutwand oder an einem Teil der Feder eine Ausnehmung oder ein Formschlusselement ausgebildet sein, in das das Spannelement oder Sperrelement in der Verriegelungsstellung eingreift. Zusammen mit der formschlüssigen Verriegelung ist es möglich, dass Querkräfte senkrecht zu zumindest einer der beiden immer gesperrten translatorischen Freiheitsgrade aufgebracht werden, um so zusätzlich zu den formschlüssigen Anteilen der Verriegelung eine Klemmwirkung bereitzustellen.

Die Längsführung kann als eine T-Führung, L-Führung, Zylinderführung oder Schwalbenschwanzführung ausgebildet sein, ganz allgemein sind für die Längsführung sämtliche Geometrien mit Hinterschneidungen geeignet, die zwei translatorische Freiheitsgrade sperren. Durch die Längsführung ist es möglich, dass das Basiselement und damit auch der Prothesenfinger in einer präzisen Ausrichtung zu dem Chassis und zueinander montiert werden können. Werden die Komponenten der Längsführung gegeneinander verklemmt, können die Fertigungstoleranzen vergleichsweise groß gewählt werden, da die Konstruktion der Längsführung unempfindlich gegenüber Maßabweichungen oder geometrischen Fehlstellungen ist.

In einer Weiterbildung der Erfindung ist vorgesehen, dass das Chassis scheibenartig ausgebildet ist und die Längsführung eine Ausrichtung aufweist, die orthogonal zu der Hauptfläche des scheibenartigen Chassis verläuft. Insbesondere wenn mehrere oder alle Prothesenfinger an dem Chassis auf diese Art und Weise gelagert sind, ergibt sich eine Erleichterung der Montage, da alle Basiselemente und damit auch die Prothesenfinger in einer gemeinsamen Fügerichtung orientiert sind. Die Orientierung des Chassis entspricht dabei im Wesentlichen der Handfläche einer natürlichen Hand, die Montagerichtung findet dann von der Handfläche zum Handrücken beziehungsweise von dem Handrücken zur Handfläche hin statt, also entweder in Dorsal-Richtung oder in Volar-Richtung.

Die Längsführung kann einen Endanschlag aufweisen, sodass die Orientierung des Basiselementes und damit auch der Prothesenfinger in Volar-Richtung oder Dorsal-Richtung eindeutig festlegbar ist. Dadurch wird ermöglicht, dass mehrere Prothesenfinger stets in der korrekten Orientierung zueinander an dem Chassis festgelegt werden können. Befindet sich die Nut der Längsführung an dem Chassis, weist die Nut nur eine Einführöffnung auf, das andere Ende der Nut ist geschlossen oder mit einer Sperre versehen, sodass nach dem vollständigen Einführung der Feder, die in diesem Beispiel an dem Basiselement ausgebildet oder angeordnet ist, das Basiselement in der korrekten Stellung befindlich ist. Alternativ ist es möglich, dass an der Feder ein Endanschlag ausgebildet ist, beispielsweise einen Querbolzen oder eine Wand, sodass die Feder nicht weiter in die Nut, die dann auch durchgängig ausgebildet sein kann, eingeschoben werden kann. Grundsätzlich ist es auch möglich, an gegenüberliegenden Enden der Nut und der Feder jeweils einen Endanschlag auszubilden oder anzuordnen, sodass die Längsführung von außen abgeschlossen oder abgedichtet ist. An den Endanschlägen können Dichtungen angeordnet sein, die das Eindringen von Schmutz verhindern oder zumindest behindern.

Eine Weiterbildung der Erfindung sieht vor, dass das Basiselement einen Lagerbock für den jeweiligen Prothesenfinger aufweist. An dem Basiselement kann eine Lagerungsstelle für den Prothesenfinger angeordnet oder ausgebildet sein, sodass eine Verschwenkung relativ zu dem Basiselement und nach der Festlegung des Basiselementes an dem Chassis auch relativ zu dem Chassis erfolgen kann. Das Basiselement und der jeweilige Prothesenfinger, der daran angeordnet ist, bilden somit ein Modul, das gemeinsam an dem Chassis festlegbar beziehungsweise von dem Chassis entfernt werden kann.

Das Sperrelement kann entlang der Längserstreckung der Längsführung verlagerbar gelagert sein, wodurch sich eine erleichterte Zugänglichkeit und leichte Markierbarkeit ergibt. Die Feder wird in die Nut der Längsführung entlang der Längserstreckung der Längsführung eingeschoben. In der gleichen Richtung erfolgt die Verlagerung des Spannelementes, was dann zu einer Klemmung oder Sperrung der Längsführung in der Einführrichtung und entgegen der Einführrichtung führt. Sind die Längsführungen senkrecht zu einer Hauptebene des Chassis orientiert ausgebildet, können die Basiselemente mehrere Fingerelemente beispielsweise von der Handinnenfläche eingeschoben und in der gleichen Orientierung das Spannelement verlagert werden. Dies vereinfacht die Montage und Demontage, da eine unmittelbare Verriegelung und Entriegelung der Befestigung des jeweils zu demontierenden oder zu befestigenden Prothesenfingers beziehungsweise Basiselementes erfolgen kann.

Das Spannelement kann als Schraube ausgebildet oder mit einer Schraube ausgebildet oder mit einer Schraube gekoppelt sein. Die Ausgestaltung als Schraube ermöglicht es, dass das Spannelement mit einfachen, herkömmlichen Werkzeugen betätigt werden kann. Das Spannelement selbst bleibt in dem Gewinde auch im demontierten Zustand, sodass es nicht verloren gehen kann. Es bedarf lediglich der Betätigung eines einzigen, standardisierten Elementes mit standardisierten Werkzeugen, ohne dass ein weiteres Material wie Unterlegscheiben, Bolzen, Stifte oder Klammern notwendig wären. Grundsätzlich ist es auch möglich, dass das Spannelement geneigt oder quer zur Längserstreckung der Längsführung orientiert ist, wodurch eine direkte formschlüssige oder klemmende Verriegelung durch das Spannelement erzielt werden kann. Sofern das Spannelement auf zumindest ein Sperrelement einwirkt, kann dieses durch die Verlagerung des als Schraube ausgebildeten Spannelementes in eine formschlüssige Verriegelungsstellung oder eine klemmende Verriegelungsstellung gebracht werden.

Dem Spannelement oder Sperrelement, sofern dies vorhanden ist, kann eine Ausnehmung zugeordnet sein, in die es in der Verriegelungsstellung eingreift. Der Eingriff kann rein formschlüssig erfolgen, beispielsweise durch Verlagerung oder Verschiebung des Spannelementes oder Sperrelementes in die Ausnehmung; alternativ oder ergänzend kann durch das Spannelement oder Sperrelement in die Ausnehmung oder auf den Träger der Ausnehmung eine Kraft ausgeübt werden, durch eine unterstützende Klemmung und gleichzeitiger Spielausgleich erfolgt. Befindet sich das Sperrelement in dem Chassis, ist die Ausnehmung in dem Basisteil ausgebildet und umgekehrt.

Vorteilhafterweise ist die Ausnehmung an einer Position in der Nut oder an der Feder der Längsführung angeordnet, in der das Basiselement an dem Chassis seine Endposition innehat. Erst wenn die Endposition erreicht ist, kann das Spannelement oder Sperrelement in die Ausnehmung eingreifen. Bei einer spielausgleichenden und selbstzentrierenden Ausgestaltung der Ausnehmung und des korrespondierenden Spannelementes beziehungsweise Sperrelementes ist es möglich, dass durch das Einführen und Einbringen des Spannelementes oder des Sperrelementes in die Ausnehmung das Basiselement an dem Chassis in seine Endposition bewegt wird. Dies kann durch eine kugelförmige oder kegelförmige oder anderweitig angeschrägte Ausgestaltung zumindest eine der beiden Verbindungspartner erfolgen, also beispielsweise durch eine kalottenförmige Ausgestaltung der Ausnehmung und eine kugelförmige Ausgestaltung des Spannelementes oder Sperrelementes. Auch bei einer keilartigen Ausgestaltung des Sperrelementes und einer rechtwinkligen Nut als Ausnehmung kann eine solche zusätzliche Spannwirkung in Längserstreckung erfolgen, um das Basiselement an dem Chassis in seine Endposition zu bewegen und gespannt in der Endposition zu halten. Ebenso kann die Ausnehmung einer Einführschräge aufweisen.

Eine Weiterbildung der Erfindung sieht vor, dass das Sperrelement über das Spannelement in der Verriegelungsstellung oder Entriegelungsstellung vorgespannt ist. Über die Vorspannung in der Verriegelungsstellung wird die Sicherung des Basiselementes in dem Chassis gewährleistet. Alternativ zu einer aktiven Verriegelung des Sperrelementes über das Spannelement ist es möglich, dass das Spannelement an dem Basiselement oder dem Chassis über ein Federelement in die Verriegelungsstellung verlagert wird, das Spannelement zur Demontage das Sperrelement in die Entriegelungsstellung bewegt. Ist das Basiselement an dem Chassis montiert, wird das Spannelement betätigt und die Blockade des Sperrelementes aufgehoben, sodass dieses durch die Rückstellkraft des Federelementes oder Elastomerelementes in die Verriegelungsstellung verlagert wird. Das Spannelement kann die Vorspannung in der Verriegelungsstellung unterstützen.

Das Sperrelement kann in Querrichtung zu einer Verlagerungsrichtung des Spannelementes verlagerbar gelagert sein, beispielsweise über Zahnradgetriebe, ein Gewinde oder über eine Verschiebung. Ist beispielsweise das Spannelement als eine Schraube ausgebildet, kann das Sperrelement mit einer Schräge oder gerundeten Kontaktfläche versehen sein, sodass bei einer Längsverlagerung der Schraube eine Querverlagerung des Sperrelementes in Querrichtung zu der Verlagerungsrichtung des Spannelementes erfolgt. Durch die Verlagerung des Sperrelementes wird dann eine formschlüssige und/oder klemmende Verriegelung der Längsführung erreicht.

Das Sperrelement kann in eine Aufnahme oder Führung verschieblich oder verlagerbar innerhalb des Chassis ausgebildet sein. Bevorzugt ist die Lagerung des Sperrelementes an dem Basiselement oder dem Chassis so ausgebildet, dass im demontierten Zustand des Basiselementes das Sperrelement nicht verloren geht. Das Sperrelement ist mit der Basis beziehungsweise dem Chassis als Modul ausgebildet.

Das Sperrelement kann als Kugel oder Kegel ausgebildet sein oder weist zumindest einen abgerundeten Bereich auf, der gegen die Feder oder die Nutwand der Längsführung drückt. Dadurch wird eine hohe Flächenpressung erzeugt oder, bei Eingriff in eine Ausnehmung, eine Selbstzentrierung und Orientierung sowie ein automatischer Spielausgleich bewirkt.

Während in bisherigen Systemen für eine Reparatur mehr oder weniger viele zusätzliche Komponenten einer Prothesenhand entfernt werden müssen, die nicht mit dem zu demontierenden Finger selbst in Verbindung stehen, ist mit der vorliegenden Erfindung die einfache Montage beziehungsweise Demontage von Prothesenfingern möglich. Bei der integrierten Ausgestaltung des Spannelementes und gegebenenfalls des Sperrelementes in dem Chassis beziehungsweise dem Basiselement müssen keine Bauteile mehr demontiert und deponiert werden, vielmehr sind sämtliche Komponenten, die zu der Verriegelung des Basiselementes an dem Chassis benötigt werden, in dem Chassis oder dem Basiselement integriert. Beispielsweise kann die Nut der Längsführung an einer Wandung des Chassis oder an einem Rand des Chassis ausgebildet oder angeordnet sein. Die Chassiswandung bildet einen Aufnahmeraum für elektronische und/oder mechanische Komponenten der Prothesenhand aus, um beispielsweise für Motoren, Energiespeicher, Steuerungseinrichtungen, Sensoren, mechanische Komponenten, Energiespeicher und dergleichen mehr. Der Hohlraum ist beispielsweise aus einem massiven Grundkörper herausgefräst oder herausgearbeitet und wird nach der Montage der mechanischen Komponenten verschlossen. Lediglich Durchführungen zur Aktuierung der Prothesenfinger sind noch vorhanden. Dies ermöglicht einen einfach Aufbau des Chassis. Eine entsprechende Konstruktion ist bei einem Chassis mit einem Rand ohne Hohlraum vorgesehen.

An dem Basiselement ist in einer Bohrung und in einem Gewinde ein Spannelement angeordnet, das ein Sperrelement so verlagert, dass der ursprünglich freie translatorische Freiheitsgrad, der zur Einführung des Basiselementes in die Nut an dem Chassis notwendig ist, gesperrt wird. Gleichzeitig wird das Basiselement in der Nut verklemmt und justiert, sodass Fertigungstoleranzen groß gewählt werden können. Das Sperrelement ist beispielsweise als eine Kugel ausgebildet, die außerhalb der Mittelachse der Gewindebohrung angeordnet ist. Als Spannelement kann eine Madenschraube mit einer Kegelspitze verwendet werden, die bei dem Eindringen der Schraube in das Gewinde das kugelförmige Sperrelement quer zur Längserstreckung der Schraube und der Längsführung verlagert und in eine kalottenförmige Ausnehmung innerhalb der Nutwandung einpresst. Somit muss zur Montage und Demontage nur eine einzige Schraube betätigt werden, weitere Bauteile sind nicht notwendig. Grundsätzlich ist auch eine umgekehrte Anordnung von Nut und Feder der Längsführung möglich, also eine Ausgestaltung der Nut in dem Basiselement und der Feder in oder an dem Chassis. Ebenso ist es möglich, das Spannelement an dem Chassis anzuordnen, entweder in der Feder oder neben der Nut.

Die Längsführung ermöglicht es, dass das Basiselement mit einer hohen Festigkeit und Stabilität an dem Chassis festgelegt werden kann. Während die übliche Befestigung über Schrauben oder Bolzen aufgrund des limitierten Bauraumes schnell an die Stabilitätsgrenzen kommt und es häufig zu Materialversagen kommt, sind über die Längsführung aufgrund der großen Flächen und der stabilen Ausgestaltung hohe Kräfte einfach aufnehmbar. Insbesondere wenn die Einführöffnung der Längsführung von der Handflächenseite möglich ist, die Verschiebung also in Dorsal-Richtung erfolgt und ein oder mehrere Endanschläge an der Nut und der Feder angeordnet oder ausgebildet sind, können die üblicherweise in Dorsal-Richtung wirkenden Kräfte sehr gut aufgenommen werden. Zug- und Druckkräfte, die in proximaler und distaler Richtung wirken, werden über die großen Flächen der Längsführung wirksam aufgenommen, sodass dort nur vergleichsweise geringe Flächenpressungen auftreten.

Bei mehreren Prothesenfingern, die entsprechend über die Längsführung an dem Chassis befestigt werden, bildet jede Fingereinheit aus Prothesenfinger und Basisteil eine Einheit, die unabhängig voneinander montiert und demontiert werden können. In dem Chassis wird aufgrund der Ausgestaltung der Längsführung mit einer geschlossenen Nut kein Loch als Durchführung zu dem Chassis benötigt.

Nachfolgend wird ein Ausführungsbeispiel der Erfindung anhand der beigefügten Figuren näher erläutert. Es zeigen:
- Figur 1 -: eine Extensionsdarstellung einer Fingereinheit mit Prothesenfinger und Basiselement sowie einem Teil eines Chassis;
- Figur 2 -: mehrere Ansichten eines montierten Prothesenfingers;
- Figur 3 -: zwei Schnittdarstellung des Details Z und gemäß B-B der Figur 2 im verriegelten Zustand;
- Figur 4 -: eine Schnittdarstellung gemäß Figur 3 im gelösten Zustand;
- Figur 5 -: eine Variante der Figur 2;
- Figur 6 -: eine Variante der Figur 3 mit zwei Sperrelementen; sowie
- Figur 7 -: eine schematische Darstellung einer Prothesenhand.

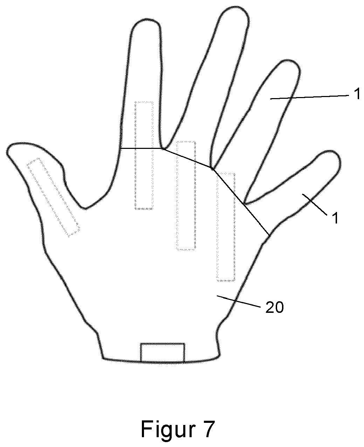

In der Figur 7 ist eine schematische Darstellung einer Prothesenhand gezeigt, bei der an einem Chassis 20, das der Handfläche einer natürlichen Hand entspricht, mehrere Prothesenfinger 1 angeordnet sind. Die Prothesenfinger 1 sind auswechselbar an dem Chassis 20 gelagert. Innerhalb des Chassis 20 oder an dem Chassis 20 können Motoren, Steuerungseinrichtungen, Getriebe und dergleichen angeordnet sein, um die Prothesenfinger 1 relativ zu dem Chassis 20 zu verlagern. Das Chassis 20 ist an einem Unterarmrohr oder einem Unterarmschaft befestigbar, insbesondere lösbar befestigbar. Elektrische Verbindungen können von dem Chassis 20 in die Prothesenfinger 1 führen, ebenso mechanische Kraftübertragungseinrichtungen, wie Hebel, Schubstangen, Zugstangen oder Kabel, Bänder, Gurte oder dergleichen.

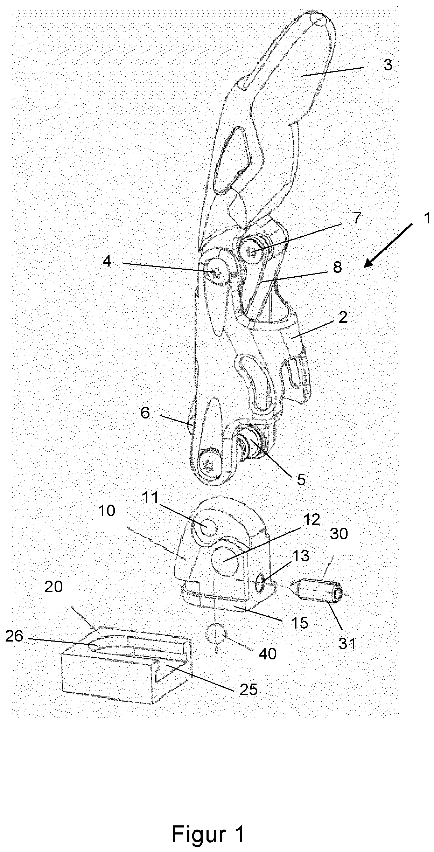

Figur 1 zeigt eine detaillierte Darstellung einer Fingereinheit aus dem Prothesenfinger 1 mit einem Basiselement 10 und eine Teilansicht des Chassis 20. Der Prothesenfinger 1 weist ein proximales Fingerglied 2 oder ein distales Fingerglied 3 auf, die über eine Schwenkachse 4 gelenkig miteinander verbunden sind. Das proximale Ende des proximalen Fingergliedes 2 ist über eine Schwenkachse 5 an einem Basiselement 10 festlegbar. Auf unterschiedlichen Seiten der Verbindungslinie zwischen den beiden Schwenkachsen 4 und 5 sind Lagerachsen 6 und 7 angeordnet, die über einen Hebel 8 miteinander gekoppelt sind. Die Lagerachse 7 an dem distalen Fingerglied 3 ist volar angeordnet, die Lagerachse 6 an dem proximalen Fingerglied 2 ist dorsal der Verbindungslinie angeordnet.

An dem Basiselement 10 ist ein Lagerbock mit zwei Bohrungen 11, 12 ausgebildet, die beabstandet zueinander angeordnet sind. Die proximale Lagerbohrung 12 dient zur Aufnahme der Schwenkachse 5, die distale, dorsal angeordnete Lagerbohrung 11 dient zur Aufnahme der Lagerachse 6. Wird das proximale Fingerglied 3 um die Lagerachse 5 in Dorsalrichtung zum Schließen der Hand verschwenkt, beispielsweise durch ein Zugmittel, das in Ausnehmungen an dem proximalen Fingerglied 2 eingreift, führt dies aufgrund des Abstandes der beiden Achsen 5, 6 zu einer Verlagerung der Lagerachse 7 relativ zu der Schwenkachse 4, sodass das distale Fingerglied 3 flektiert wird. Bei einer umgekehrten Bewegung erfolgt eine Extension des distalen Fingergliedes 3.

An dem Basiselement 10 ist zudem eine Feder 15 als Teil einer Längsführung ausgebildet. Die Feder 15 weist einen im Wesentlichen T-förmigen Querschnitt auf und ist an dem proximalen Ende des Basiselementes 10 ausgebildet. Eine entsprechend geformte Platte könnte als alternative Ausgestaltung an der Unterseite des Basiselementes 10 befestigt sein, um eine solche Feder 15 auszubilden. Die Feder 15 ist ausgebildet, um in eine Nut 25 der Basis 20 eingeführt zu werden. Der Querschnitt der Nut 25 entspricht dem Querschnitt der Feder 15. An dem dorsalen Ende der Nut 25 ist ein Endanschlag 26 ausgebildet, sodass das Basiselement 10 nur bis dorthin eingeschoben werden kann. Von oben gesehen ist die Nut 25 am dorsalen Ende gerundet ausgebildet, andere Formgebungen sind ebenfalls möglich. Ebenfalls sind abweichende Querschnittsformen der Längsführung möglich. Neben einer Schwalbenschwanzführung oder einer L-förmigen Führung können alle Längsführungen vorgesehen sein, die zwei translatorische Freiheitgrade und vorteilhafterweise alle rotatorischen Freiheitsgrade zwischen dem Chassis 20 und dem Basiselement 10 blockieren.

Innerhalb des Basiselementes 10 ist eine Bohrung 13 mit einem Innengewinde ausgebildet, in das ein Spannelement 30 in Gestalt einer Madenschraube mit einem Außengewinde einschraubbar ist. Die Spitze des Spannelementes 30 ist konisch ausgebildet und wirkt auf ein Sperrelement 40, das innerhalb einer weiteren Bohrung oder Führung in dem Basiselement 10 gelagert ist. Die Ausnehmung kann als Querbohrung zu der Bohrung 13 in dem Basiselement 10 ausgebildet sein. Um ein Herausfallen des Sperrelementes 40, das im dargestellten Ausführungsbeispiel als Kugel ausgebildet ist, zu verhindern, kann eine Fettfüllung oder eine andere Sicherungseinrichtung eingearbeitet oder an dem Basiselement 10 angeordnet sein.

Auf der Unterseite der Nut 25 kann eine korrespondierende Ausnehmung zur Aufnahme des Sperrelementes 40 ausgebildet sein, beispielsweise eine kalottenförmige oder konische Ausnehmung.

In dem dargestellten Ausführungsbeispiel ist die Nut 25 aus einem Vollmaterial des Chassis 20 herausgefräst oder herausgearbeitet. Alternativ kann die Nut 25 auch mehrteilig ausgebildet sein, indem eine entsprechend geformte Platte mit Ausnehmungen über einer Basisnut angeordnet wird.

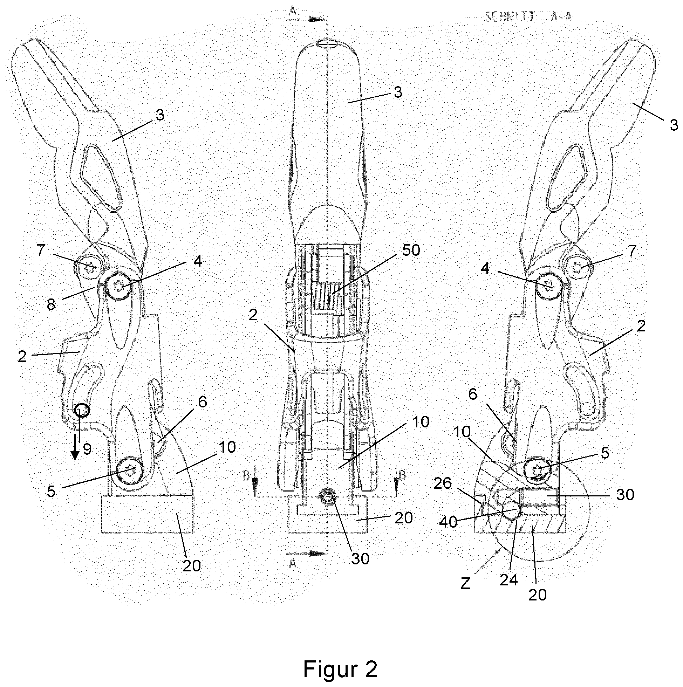

In der Figur 2 sind drei Darstellungen eines montierten Prothesenfingers 1 an dem Chassis 20 gezeigt. Die linke Darstellung zeigt eine Seitenansicht mit dem Chassis 20 und dem Basiselement 10, das in der Nut befestigt ist. Wird auf ein Kopplungselement 9, das in eine entsprechende Führung innerhalb des proximalen Fingergliedes 2 angeordnet ist, eine Zugkraft in Richtung des Pfeiles ausgeübt, verschwenkt das proximale Fingerglied 2 um die Schwenkachse 5. Der Hebel 8 bleibt an der Lagerachse 7 fixiert und dreht gegen den Uhrzeigersinn zusammen mit dem proximalen Fingerglied 2, allerdings nur um die proximale Lagerachse 6. Die Schwenkachse 4 ebenso wie die Lagerachse 7 verschwenken ebenfalls entgegen der Uhrzeigerrichtung, das distale Fingerglied 3 schwenkt zudem aufgrund der starren Kopplung zwischen den beiden Lagerachsen 6, 7, die eine verschwenkbare Verlagerung ermöglichen, um die Schwenkachse 4. Eine Rückstellung kann über eine entgegengesetzte Verlagerung des proximalen Fingergliedes 2 oder eine Feder erfolgen. Ein Antrieb kann an der dorsalen Seite des Chassis 20 angeordnet sein, gegebenenfalls in einer Ausnehmung, die durch eine Chassiswand begrenzt wird.

In der mittleren Darstellung der Figur 2 ist eine Dorsalansicht des Prothesenfingers gezeigt. Das Chassis 20 ist nur teilweise dargestellt. Es ist zu erkennen, dass das Basiselement 10 innerhalb der Nut 25 in dem Chassis 20 eingeführt ist. Das Basiselement 10 weist einen im Wesentlichen T-förmigen Querschnitt auf, sodass es in die korrespondierend ausgebildete Nut 25 eingeführt werden kann. Nach dem Einführen, bevor das Spannelement 30 aktiviert und das Sperrelement 40 eine Verriegelung bewirkt, ist das Basiselement 10 bereits in zwei translatorischen Freiheitsgraden eingeschränkt, die Bewegungen senkrecht zu der Einführrichtung sind gesperrt. Auf der Innenseite des Prothesenfingers 1 ist eine Feder 50 zu erkennen, die eine Extension des distalen Fingergliedes 3 relativ zu dem proximalen Fingerglied 2 in Extensionsrichtung bewirkt. Wird keine Zugkraft mehr durch das Kopplungselement 9 ausgeübt, wird durch die Rückstellkraft der Feder 50 eine Extension und damit eine Rückstellung in die Ausgangsposition bewirkt.

In der rechten Darstellung der Figur 2 ist eine Schnittdarstellung gemäß A-A gezeigt. Insbesondere der Einzelheit Z ist zu entnehmen, dass das Basiselement 10 mit dem über das Chassis 20 distal hinausragenden Lagerbock für die beiden Achsen 5, 6 vollständig, bis zu einem Endanschlag 26, in der Nut 25 eingeführt ist. Das Spannelement 30 ist in das Gewinde der Bohrung 13 eingeschraubt und wirkt auf das Sperrelement 40, das auf den Boden der Nut 25 in eine Ausnehmung 24 gepresst wird. Die Ausnehmung 24 kann kalottenförmig oder konisch ausgebildet sind, sodass bei einer nicht vollständigen Einführbewegung des Basiselementes 10 sich eine automatische Selbstzentrierung und Selbstausrichtung innerhalb des Chassis 20 einstellt. Durch das Einschrauben des Spannelementes 30 in Dorsalrichtung wird das Sperrelement 40 in Gestalt der Kugel in Proximalrichtung in die Ausnehmung 24 gedrückt. Dadurch wird die Feder 15 innerhalb der Nut 25 formschlüssig verriegelt und darüber hinaus eine Klemmung an der Oberkante der T-förmigen Nut erreicht. Die Basis 10 und damit der Prothesenfinger 1 sind somit spielfrei und stabil innerhalb der Nut 25 an dem Chassis 20 gelagert. Es können hohe Haltekräfte durch diese Art der Verriegelung erreicht werden, da große Anlageflächen vorhanden sind, um Druckkräfte und Zugkräfte aufzunehmen. Eine Verlagerung in Dorsalrichtung, also in Richtung auf den Handrücken, wird durch den massiven Endanschlag 26 verhindert, sodass sehr hohe Druckkräfte in Dorsalrichtung aufgenommen werden können. Die Druckkräfte in Volarrichtung sind in der Regel wesentlich geringer und führen zu einem Einknicken der Prothesenfinger, was durch die Langlochführung für das Kopplungselement 9 angedeutet ist.

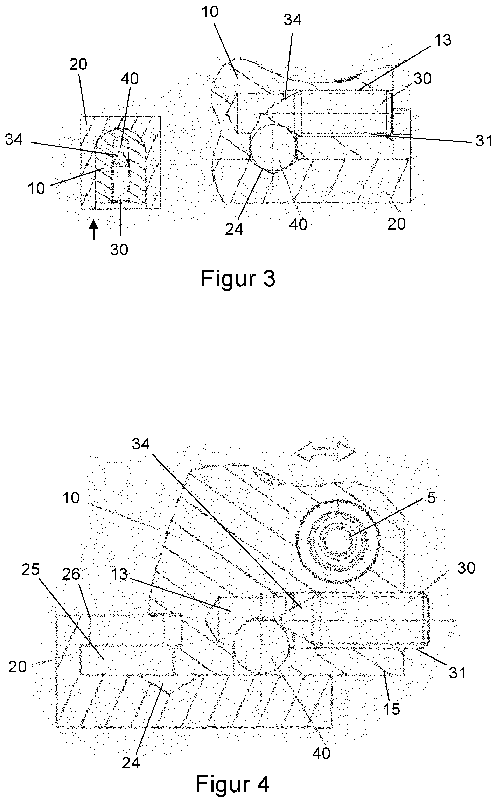

In der Figur 3 sind in der linken Darstellung der Schnitt B-B und in der rechten Darstellung die Einzelheit Z dargestellt. In der linken Darstellung ist zu erkennen, dass das Spannelement 30 auf das Sperrelement 40 wirkt und dieses aufgrund der konischen Spitze 34 nach unten in Richtung auf die geschlossene Bodenfläche der Nut 25 des Basisteils 20 drückt. Sowohl das Spannelement 30 als auch das Basiselement 10 werden in dieselbe Richtung verlagert, sodass eine Montage sehr einfach von der Handflächeninnenseite oder von volar in Dorsalrichtung stattfinden kann.

In der rechten Darstellung der Figur 3 ist in einer Schnittdarstellung zu erkennen, dass das kugelförmige Sperrelement 40 durch die kegelige Spitze 34 des Sperrelementes 30 in die konische Ausnehmung 24 gepresst wird. Das Spannelement 30 ist mit einem Außengewinde 31 versehen, das in das Innengewinde der Bohrung 13 eingreift. Selbst bei einem teilweisen Herausschrauben der Schraube bzw. des Sperrelementes 30 verbleibt dieses weiterhin dauerhaft an dem Basiselement 10. Das Sperrelement 40 kann, wie oben ausgeführt, in einer Fettlagerung, durch eine magnetische Kraft oder durch einen Haltering an der Unterseite oder eine andere geeignete Halteeinrichtung innerhalb der nach unten führenden Bohrung zur Aufnahme des Sperrelementes 40 gehalten und gegen ein Herausfallen gesichert werden.

In der Figur 4 ist die Anordnung mit dem Basiselement 10 und dem Chassis 20 in einer Schnittdarstellung gemäß der Einzelheit Z in einem nicht montierten Zustand gezeigt. Das Spannelement 30 ist teilweise aus dem Basiselement 10 herausgeschraubt, das Sperrelement 40 ist innerhalb der Bohrung 13 aufgenommen und ragt nicht über die Unterseite der Feder 15 hinaus. Das Basiselement 10 kann innerhalb der Nut 25 in der Einführ- und Ausführrichtung, die durch den Doppelpfeil angedeutet sind, bewegt werden. Der nicht dargestellte Prothesenfinger ist über die Schwenkachse 5 bereits an dem Basiselement 10 fertig montiert und kann zusammen mit diesem als Modul an dem Chassis 20 festgelegt werden. Zur Festlegung wird das Basiselement 10 vollständig bis zu dem Endanschlag 26 eingeführt. Die Querbohrung für das Sperrelement 40 liegt dann im Wesentlichen korrespondierend zu der Ausnehmung 24. Anschließend wird das Spannelement 30 eingeschraubt, der Konus 34 drückt das Sperrelement 40 nach unten in die Ausnehmung 24 und führt zu einer formschlüssigen und klemmenden Verriegelung des Basiselementes 10 innerhalb des Chassis 20. Zum Lösen wird das als Madenschraube ausgebildete Spannelement 30 herausgeschraubt, sodass das Sperrelement in die Bohrung 13 hinein bewegt werden kann, wodurch die Klemmwirkung und die formschlüssige Verriegelung aufgehoben wird. Der Prothesenfinger 1 kann dann zusammen mit dem Basiselement 10, gegebenenfalls nach Entkopplung des Kopplungselementes 9 von dem Chassis 20 abgenommen werden.

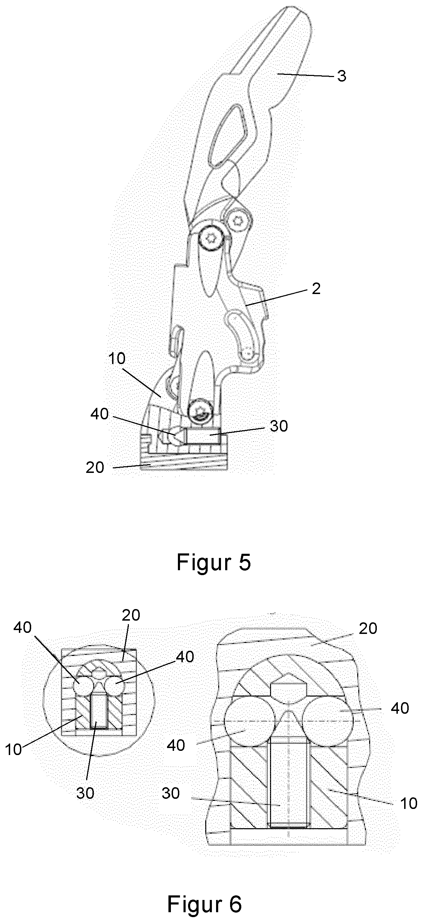

In der Figur 5 ist eine Variante der Befestigung dargestellt, wobei die Figur 5 der rechten Darstellung der Figur 2 entspricht. Der grundsätzliche Aufbau ist der gleiche, alternativ zu einer Verklemmung mit der Basis der Nut 25 ist das Sperrelement 40 in einer Ebene parallel zu der Basisfläche der Nut 25 angeordnet und wirkt somit auf eine Seitenfläche der Nut 25. Bei nur einem Sperrelement 40 wird die Feder 15 in die entgegengesetzte Richtung gedrückt. Sind zwei Sperrelemente vorhanden, erfolgt eine mittige Selbstzentrierung.

In der Figur 6 ist die Ausführungsform mit zwei Sperrelementen 40 gezeigt, die linke Darstellung entspricht der linken Darstellung der Figur 4, dementsprechend entspricht die rechte Darstellung der rechten Darstellung der Figur 4. Es ist zu erkennen, dass zwei Sperrelemente 40 einander gegenüberliegend in einer Querbohrung innerhalb des Basiselementes 10 angeordnet sind. Die Querbohrung verläuft orthogonal zu der Bohrung 13 und zu der Einführrichtung des Spannelementes 30. Die konische Spitze 34 wirkt auf beide Sperrelemente 40 und drückt diese nach außen in Ausnehmungen oder nur gegen die Seitenwand der Nut 25.

Alternativ ist es möglich, dass ein Sperrelement in die Bodenfläche oder in die Basisfläche der Nut 25 hineingepresst und das andere Sperrelement 40 an eine Seitenwand der Nut 25. Grundsätzlich ist es ebenfalls möglich, dass drei Sperrelemente 40 vorgesehen sind, zwei in Wechselwirkung mit den Seitenflächen der Nut, eines mit der Bodenfläche.

Grundsätzlich ist es ebenfalls möglich, dass das Sperrelement und das Spannelement in dem Chassis angeordnet sind und auf die Feder in dem Basiselement einwirken. In der Feder kann dann eine Ausnehmung angeordnet sein. Grundsätzlich ist es auch möglich, dass das Spannelement direkt eine Verklemmung und/oder formschlüssige Verriegelung bewirkt. Ebenso ist eine kinematische Umkehr möglich, also die Ausgestaltung der Nut innerhalb des Basiselementes und der Feder innerhalb oder an dem Chassis.

## Patentansprüche

1. Prothesenhand mit einem Chassis (20) und zumindest einem Basiselement (10) zur Befestigung eines Prothesenfingers an dem Chassis (20), **dadurch gekennzeichnet, dass** an dem Chassis (20) eine Längsführung für das Basiselement (10) angeordnet oder ausgebildet ist, in der das Basiselement (10) gelagert ist, wobei die Längsführung zwei translatorische Freiheitsgrade sperrt und der Längsführung ein Spannelement (30) zugeordnet ist, das zwischen einer Verriegelungsstellung und einer Entriegelungsstellung verlagerbar in oder an dem Chassis (20) oder dem Basiselement (10) gelagert ist und eine Sperrung des dritten translatorischen Freiheitsgrades in der Verriegelungsstellung bewirkt.

2. Prothesenhand nach Anspruch 1, **dadurch gekennzeichnet, dass** das Spannelement (30) auf zumindest ein Sperrelement (40) einwirkt und dieses aus einer Entriegelungsstellung in eine Verriegelungsstellung oder umgekehrt bringt.

3. Prothesenhand nach Anspruch 1 oder 2, **dadurch gekennzeichnet, dass** das Spannelement (30) oder das Sperrelement (40) das Basiselement (10) in der Längsführung klemmt und/oder formschlüssig verriegelt.

4. Prothesenhand nach einem der voranstehenden Ansprüche, **dadurch gekennzeichnet, dass** die Längsführung als eine T-Führung, L-Führung, Zylinderführung oder Schwalbenschwanzführung ausgebildet ist.

5. Prothesenhand nach einem der voranstehenden Ansprüche, **dadurch gekennzeichnet, dass** das Chassis (20) scheibenartig ausgebildet ist und die Längsführung orthogonal zu der Hauptfläche ausgerichtet ist.

6. Prothesenhand nach einem der voranstehenden Ansprüche, **dadurch gekennzeichnet, dass** die Längsführung zumindest einen Endanschlag (26) aufweist.

7. Prothesenhand nach einem der voranstehenden Ansprüche, **dadurch gekennzeichnet, dass** das Basiselement (10) einen Lagerbock für den jeweiligen Prothesenfinger aufweist.

8. Prothesenhand nach einem der voranstehenden Ansprüche, **dadurch gekennzeichnet, dass** das Spannelement (30) entlang der Längserstreckung der Längsführung verlagerbar gelagert ist.

9. Prothesenhand nach einem der voranstehenden Ansprüche, **dadurch gekennzeichnet, dass** das Spannelement (30) als Schraube ausgebildet oder mit einer Schraube gekoppelt ist.

10. Prothesenhand nach einem der voranstehenden Ansprüche, **dadurch gekennzeichnet, dass** dem Spann- oder Sperrelement (30, 40) eine Ausnehmung (24) zugeordnet ist, in die es in der Verriegelungsstellung eingreift.

11. Prothesenhand nach Anspruch 10, **dadurch gekennzeichnet, dass** die Ausnehmung (24) an einer Position angeordnet ist, in der das Basiselement (10) an dem Chassis (20) seine Endposition innehat.

12. Prothesenhand nach einem der Ansprüche 2 bis 11, **dadurch gekennzeichnet, dass** das Sperrelement (40) über das Spannelement (30) in der Verriegelungsstellung oder Entriegelungsstellung vorgespannt ist.

13. Prothesenhand nach einem der Ansprüche2 bis 12, **dadurch gekennzeichnet, dass** das Sperrelement (40) in Querrichtung zu einer Verlagerungsrichtung des Spannelementes (30) verlagerbar gelagert ist.

14. Prothesenhand nach einem der Ansprüche 2 bis 13, **dadurch gekennzeichnet, dass** das Sperrelement (40) in einer Aufnahme oder Führung verschieblich oder verlagerbar gelagert ist

15. Prothesenhand nach einem der voranstehenden Ansprüche, **dadurch gekennzeichnet, dass** das Sperrelement (40) als Kugel oder Kegel ausgebildet ist oder zumindest einen abgerundeten Bereich aufweist.

## Claims

1. Prosthetic hand with a chassis (20) and at least one base element (10) for fastening a prosthetic finger to the chassis (20), **characterized in that** a longitudinal guide for the base element (10) is arranged or formed on the chassis (20), in which longitudinal guide the base element (10) is mounted, wherein the longitudinal guide blocks two translatory degrees of freedom and a clamping element (30) is associated with the longitudinal guide, which clamping element is displaceably mounted in or on the chassis (20) or the base element (10) between a locking position and an unlocking position and effects a blocking of the third translatory degree of freedom in the locking position.

2. Prosthetic hand according to claim 1, **characterized in that** the clamping element (30) acts on at least one locking element (40) and brings the latter from an unlocking position into a locking position or vice versa.

3. Prosthetic hand according to claim 1 or 2, **characterized in that** the clamping element (30) or the locking element (40) clamps and/or positively locks the base element (10) in the longitudinal guide.

4. Prosthetic hand according to one of the preceding claims, **characterized in that** the longitudinal guide is designed as a T-guide, L-guide, cylinder guide or dovetail guide.

5. Prosthetic hand according to one of the preceding claims, **characterized in that** the chassis (20) is disc-shaped and the longitudinal guide is oriented orthogonally to the main surface.

6. Prosthetic hand according to one of the preceding claims, **characterized in that** the longitudinal guide has at least one end stop (26).

7. Prosthetic hand according to one of the preceding claims, **characterized in that** the base element (10) has a bearing block for the respective prosthetic finger.

8. Prosthetic hand according to one of the preceding claims, **characterized in that** the clamping element (30) is displaceably mounted along the longitudinal extension of the longitudinal guide.

9. Prosthetic hand according to one of the preceding claims, **characterized in that** the clamping element (30) is designed as a screw or is coupled to a screw.

10. Prosthetic hand according to one of the preceding claims, **characterized in that** the clamping or locking element (30, 40) is assigned a recess (24) in which it engages in the locking position.

11. Prosthetic hand according to claim 10, **characterized in that** the recess (24) is arranged at a position in which the base element (10) has its end position on the chassis (20).

12. Prosthetic hand according to any one of claims 2 to 11, **characterized in that** the locking element (40) is biased via the clamping element (30) in the locking position or unlocking position.

13. Prosthetic hand according to one of the claims 2 to 12, **characterized in that** the locking element (40) is mounted displaceably in the transverse direction to a displacement direction of the clamping element (30).

14. Prosthetic hand according to one of claims 2 to 13, **characterized in that** the locking element (40) is mounted displaceably or displaceably in a receptacle or guide.

15. Prosthetic hand according to one of the preceding claims, **characterized in that** the locking element (40) is designed as a ball or cone or has at least one rounded region.

## Revendications

1. Main prothétique comprenant un châssis (20) et au moins un élément de base (10) destiné à fixer un doigt prothétique au châssis (20),
**caractérisée en ce qu'**un guidage longitudinal pour l'élément de base (10) est disposé ou réalisé sur le châssis (20), guidage dans lequel l'élément de base (10) est monté, le guidage longitudinal bloquant deux degrés de liberté de translation, et un élément de serrage (30) étant associé au guidage longitudinal, qui est monté dans ou sur le châssis (20) ou l'élément de base (10) de manière à pouvoir être déplacé entre une position de verrouillage et une position de déverrouillage et qui provoque un blocage du troisième degré de liberté de translation dans la position de verrouillage.

2. Main prothétique selon la revendication 1,
**caractérisée en ce que** l'élément de serrage (30) agit sur au moins un élément de blocage (40) et le fait passer d'une position de déverrouillage à une position de verrouillage ou inversement.

3. Main prothétique selon la revendication 1 ou 2,
**caractérisée en ce que** l'élément de serrage (30) ou l'élément de blocage (40) serre l'élément de base (10) dans le guidage longitudinal et/ou le verrouille par coopération de formes.

4. Main prothétique selon l'une des revendications précédentes,
**caractérisée en ce que** le guidage longitudinal est conçu comme un guidage en T, un guidage en L, un guidage cylindrique ou un guidage en queue d'aronde.

5. Main prothétique selon l'une des revendications précédentes,
**caractérisée en ce que** le châssis (20) est réalisé en forme de disque, et le guidage longitudinal est orienté orthogonalement à la surface principale.

6. Main prothétique selon l'une des revendications précédentes,
**caractérisée en ce que** le guidage longitudinal présente au moins une butée de fin de course (26).

7. Main prothétique selon l'une des revendications précédentes,
**caractérisée en ce que** l'élément de base (10) présente un support de palier pour le doigt prothétique correspondant.

8. Main prothétique selon l'une des revendications précédentes,
**caractérisée en ce que** l'élément de serrage (30) est monté de manière à pouvoir être déplacé le long de l'extension longitudinale du guidage longitudinal.

9. Main prothétique selon l'une des revendications précédentes,
**caractérisée en ce que** l'élément de serrage (30) est conçu comme une vis ou est couplé à une vis.

10. Main prothétique selon l'une des revendications précédentes,
**caractérisée en ce qu'**un évidement (24) est associé à l'élément de serrage ou de blocage (30, 40), dans lequel il s'engage dans la position de verrouillage.

11. Main prothétique selon la revendication 10,
**caractérisée en ce que** l'évidement (24) est disposé dans une position dans laquelle l'élément de base (10) occupe sa position de fin de course sur le châssis (20).

12. Main prothétique selon l'une des revendications 2 à 11,
**caractérisée en ce que** l'élément de blocage (40) est précontraint dans la position de verrouillage ou dans la position de déverrouillage par l'intermédiaire de l'élément de serrage (30).

13. Main prothétique selon l'une des revendications 2 à 12,
**caractérisée en ce que** l'élément de blocage (40) est monté de manière à pouvoir être déplacé dans une direction transversale par rapport à une direction de déplacement de l'élément de serrage (30).

14. Main prothétique selon l'une des revendications 2 à 13,
**caractérisée en ce que** l'élément de blocage (40) est monté de manière à pouvoir coulisser ou se déplacer dans un logement ou un guidage.

15. Main prothétique selon l'une des revendications précédentes,
**caractérisée en ce que** l'élément de blocage (40) est réalisé sous forme de sphère ou de cône ou présente au moins une zone arrondie.
